# EUROPÄISCHE PATENTSCHRIFT

(11) **EP 1 142 536 B1**
(45) Veröffentlichungstag und Bekanntmachung des Hinweises auf die Patenterteilung: **31.07.2002**
(21) Anmeldenummer: 00107088.7
(22) Anmeldetag: 05.04.2000
(51) Int. Cl.: A61B 19/00

(54) **Referenzierung eines Patienten in einem medizinischen Navigationssystem mittels aufgestrahlter Lichtpunkte**
Patient referencing in a medical navigation system using projected light points
Référencement d'un patient dans un système de navigation médical utilisant des points lumineux projetés

(43) Veröffentlichungstag der Anmeldung: 10.10.2001
(73) Patentinhaber: BrainLAB AG, 85551 Kirchheim/Heimstetten (DE)
(72) Erfinder: Vilsmeier, Stefan, 6330 Kufstein (AT)
(74) Vertreter: Rögner, Jürgen

(56) Entgegenhaltungen:
- WO-A-91/04711
- WO-A-99/38449
- DE-A- 19 639 615
- US-A- 4 597 380
- US-A- 5 851 183
- US-A- 6 006 126

## Beschreibung

Die vorliegende Erfindung betrifft ein Verfahren und eine Vorrichtung zur Referenzierung bzw. Registrierung eines Patienten bzw. eines Körperteils eines Patienten in einem kameragestützten medizinischen Navigationssystem.

Chirurgische Eingriffe oder Bestrahlungsbehandlungen werden in heutiger Zeit immer öfter mit Hilfe sogenannter Navigations- oder Trackingsysteme durchgeführt. Hierbei werden Patientendaten, die mit einem bildgebenden Verfahren ermittelt wurden, beispielsweise einer Computer- oder Kernspintomographie, dazu verwendet, dem behandelnden Arzt mittels einer Bildschirmausgabe aufzuzeigen, wo sich sein Behandlungswerkzeug momentan befindet. Ein Anwendungsfall besteht beispielsweise darin, die Position der Spitze eines Instrumentes im Inneren eines zu behandelnden Körperteils aufzuzeigen, um dadurch äußerst exakt an den zu behandelnden Stellen operieren zu können.

Damit eine solches Navigationssystem funktionieren kann, muss die momentane Lage des Patienten bzw. des zu behandelnden Körperteils vor Ort bei der Behandlung bekannt sein. Diese aktuellen Positionsdaten können dann den Daten aus dem bildgebenden Verfahren, beispielsweise den Daten aus einer eine gewisse Zeit vor der Behandlung erstellten Computertomogräphie, zugeordnet werden. Nach dieser Zuordnung kann dann die bildunterstützte Behandlung beginnen.

Gemäß dem Stand der Technik wird die vorgenannte Zuordnung mit Hilfe von Markierungen, dass heißt mit Hilfe von künstlichen oder natürlichen Landmarken am Patienten realisiert. So schlägt beispielsweise das deutsche Patent Nr. 196 39 615 vor, dem Patienten vor einer Tomographieerfassung künstliche Marker auf die Haut aufzukleben, wobei dabei solche Marker verwendet werden, die im Tomographiebild sichtbar sind. Nach der Tomographie wird der Patient in den Operationssaal gebracht. Bei Beginn der Behandlung erfolgt dann die Referenzierung des Patienten bzw. seines Körperteils dadurch, dass die an ihm angebrachten Marker mit einem im Navigationssystem verfolgbaren Zeigewerkzeug angefahren werden, um dem unterstützenden Computersystem ihre aktuelle Position bekannt zu machen. Wenn die Position der Marker bekannt ist, kann computerunterstützt nunmehr auch die Position aller anderen Punkte aus dem Tomographiedatensatz in der aktuellen Lage des Patienten ermittelt werden, und die navigationsunterstützte Behandlung kann beginnen.

Die Verwendung solcher von außen aufgebrachter Marker auf der Hautoberfläche birgt aber einige Nachteile. Zunächst einmal ist die Hautoberfläche leicht verschiebbar und solche Verschiebungen sorgen für Ungenauigkeiten bei der Referenzierung. Insbesondere beim Anfahren der Marker mit dem Zeigewerkzeug können sich leicht Hautverschiebungen einstellen.

Es können aber nicht allzu viele künstliche Marker aufgesetzt werden, um solche Ungenauigkeiten zu kompensieren, da dies die Referenzierung unnötig verlängern werden. Invasive Lösungen, die Marker beispielsweise an der Knochensubstanz unter der Haut festlegen, sind für Patienten sehr unangenehm, während natürliche Landmarken wie zum Beispiel die Nasenwurzel, oftmals nicht sehr positionsgenau referenziert werden können.

Ein weiterer Nachteil der oben genannten Methode zeigt sich vor allem dann, wenn die Behandlung nicht unmittelbar nach der Tomographie durchgeführt wird. So können sich beispielsweise über Nacht einige der Marker lösen, was zu größeren Schwierigkeiten bei der Referenzierung führen kann. Ein besonders nachteiliger Fall tritt dann auf, wenn die gelösten Markierungen von dem Patienten selbst wieder an einer anderen Stelle aufgebracht werden; dies kann sogar zu Fehlbehandlungen führen.

Ein weiteres Navigationssystem, das auf der Bereitstellung von Referenzmarkern beruht ist aus der US-A-5,383,454 bekannt. Dabei werden aktiv abstrahlende, separat bereitgestellte Marker verwendet, was ebenfalls zu den oben aufgeführten Nachteilen führt.

Aus der US-A-6,006,126 ist eine Vorrichtung gemäß dem Oberbegriff des Patentanspruchs 10 bekannt. Es wird vorgeschlagen, mittels eines Laserscanners bzw. eines optischen Scanners Lichtmuster auf dem Kopf eines Patienten zu erzeugen. Laserscanner sind jedoch sehr sperrige und unflexible Geräte. Sie werden einmal an einem bestimmten Ort aufgestellt und bleiben dann dort, wodurch die Notwendigkeit entsteht, den Patienten jedes Mal in den Strahlbereich des Scanners zu bringen, wenn mit solchen Geräten eine Referenzzierung bzw. Registrierung durchgeführt werden soll. Mit einem solchen stationären Laserscanner ist es außerdem nicht möglich, ein Lichtmuster auf Bereiche des Patientenkopfes aufzubringen und abzuscannen, deren Oberfläche im Winkel von 90° oder mehr zur Strahlrichtung liegt, also Bereiche am Patientenkopf die in Strahlrichtung "im Schatten" liegen. Um solche Bereiche mit einem Lichtmuster zu versehen bzw. abzuscannen, müsste der Patient gedreht werden, und dies bringt wiederum Schwierigkeiten durch Verschiebungen in der Gesamtregistrierung mit sich.

Aus der US-A-5,851,183 ist es bekannt, einen optischen Scanner zu verwenden, um den Stirnbereich des Patienten durch einen Scanvorgang zu erfassen. Solche rein optisch erfassenden Scanner, mit denen die Oberfläche eines Gegenstandes abgetastet werden soll, benötigen eine sehr komplizierte Erfassungssoftware und erfordern einen so hohen Rechenaufwand, das ihr Einsatz in der Praxis kaum möglich ist. Außerdem muss zur exakten Referenzzierung gemäß der US-A-5,851,183 zusätzlich die Position eines an einer Kopfhalterung befestigten Bogens mit erfasst werden.

Insgesamt ist zur Verwendung solcher optischer oder Laserscanner anzumerken, dass es sich dabei um höchst unflexible und teure Geräte handelt.

Es ist die Aufgabe der vorliegenden Erfindung, ein Verfahren und eine Vorrichtung zur Referenzierung eines Patienten bzw. eines Körperteils eines Patienten in einem kameragestützten, medizinischen Navigationssystem bereitzustellen, welche die Nachteile überwindet, die oben hinsichtlich des Standes der Technik aufgezeigt wurden. Insbesondere soll eine exakte und störungsunanfällige Referenzierung ermöglicht werden, die in einfacher und unaufwändiger Weise durchgeführt werden kann.

Diese Aufgabe wird erfindungsgemäß durch ein Verfahren zur Referenzierung bzw. Registrierung eines Patienten bzw. eines Körperteils eines Patienten in einem kameragestützten, medizinischen Navigationssystem mit den folgenden Schritten gelöst:
- das zu referenzierende Körperteil des Patienten wird in den Erfassungsbereich eines durch mindestens zwei Kameras unterstützten Navigationssystems gebracht, welches computergestützt die dreidimensionalen Raumpositionen von Lichtmarkierungen erfasst,
- mittels eines Lichtstrahles aus einem Handlichtstrahler werden auf der Oberfläche des zu referenzierenden Körperteils Lichtmarkierungen erzeugt, deren dreidimensionale Position von dem kameragestützten Navigationssystem bestimmt wird, und
- mittels der Positionsdaten für die Lichtmarkierungen wird die Raumlage der Oberfläche des zu referenzierenden Körperteils bestimmt.

Mit anderen Worten geht die vorliegende Erfindung davon ab, separate Markierungen an der Oberfläche des Patientenkörperteils anzubringen, sondern erzeugt diese Markierungen einfach durch das Aufstrahlen von Licht auf die Oberfläche (Hautoberfläche, Knochenoberfläche). Durch die Bestrahlung mit Licht wird auf der Oberfläche ein Lichtpunkt erzeugt, der für die Kameras ebenso sichtbar ist, wie die Abstrahlung einer Markierung. Fährt man nun mit diesem Lichtstrahl die Oberfläche ab, so werden praktisch eine Vielzahl von Lichtmarkierungen erzeugt, und die Zusammensetzung dieser jeweils als dreidimensionale Lichtpunkte im Raum bekannten Lichtmarkierungen ergibt eine der Oberfläche des zu behandelnden Körperteils zugeordnete Punktemenge oder Punktewolke. Theoretisch genügt die Erfassung von drei Punkten; stabiler wird das Verfahren durch die Erzeugung von mehr Punkten. Mit einer ausreichenden Anzahl dieser Punkte erhält man genügend Informationen, um die Oberfläche sehr genau räumlich zuordnen zu können. Versuche haben ergeben, dass schon eine geringe Anzahl (etwa 20) auf diese Weise erzeugter Lichtmarkierungen genügen, um die Raumlage des Körperteils mit guter Genauigkeit feststellen zu können.

Generell gilt, dass solche Patientenbereiche mit dem Lichtstrahl angestrahlt werden, welche sich in dieser Form im entsprechenden Datensatz des Patienten wiederfinden lassen. Damit ist die Referenzierung bzw. Registrierung auch dann möglich, wenn Patienten ohne jegliche Zusatzteile durch ein bildgebendes Verfahren gescannt werden und dann bei der Referenzierung bzw. der Registrierung schon solche Zusatzteile (z. B. Schläuche in Mund und Nase) aufweisen.

Der Vorteil der vorliegenden Erfindung liegt damit neben der erreichbaren großen Genauigkeit insbesondere darin, dass alle Probleme, welche mit separat angebrachten Markern entstehen, überwunden werden. Die Referenzierung leidet nicht mehr unter möglicherweise versetzten, verschobenen oder entfernten Markern. Die erfindungsgemäße Referenzierung bringt praktisch keinerlei unangenehme Nebeneffekte fiir den Patienten mit sich, wie etwa Störungen durch aufgeklebte oder gar invasiv befestigte Marker. Einzelne falsche Lichtmarkierungen bzw. Reflexe, z. B. "Ausreißer" wie an anderen als der gewünschten Oberfläche reflektierte Strahlen, können ohne weiteres rechnerisch berichtigt werden.

Ein weiterer großer Vorteil der vorliegenden Erfindung liegt darin, dass, weil keine separaten Markierungen angebracht werden müssen, die Behandlung in großem Umfang zeitlich von der Tomographieerfassung entkoppelt werden kann. Da sich die Hautoberfläche des Patienten über längere Zeit nicht wesentlich ändern wird, ist es somit möglich, den Patienten auch einige Tage nach der Tomographieerfassung noch exakt zu referenzieren, und es ist dabei nicht notwendig, dass der Patient über diese lange Zeitspanne Markierungen an der Hautoberfläche behält.

Weiterhin vorteilhaft wirkt sich die Erfindung schon bei der Scan-Erfassung des Patienten aus. Bisher werden spezielle Scans (Navigationsscans) durchgeführt, d. h. Scans, die CT- oder MR-sichtbare Marker enthalten. Die Erfindung ermöglicht es nun, da Scans ohne Marker durchgeführt werden, zum Einen die Scans zeitlich von der Operation zu entkoppeln, aber zum Anderen auch beliebige Scans zu Navigationszwecken zu benutzen. Stellt also ein Arzt vor der Operation fest, dass ein weiterer, bereits vor einigen Wochen durchgeführter CT-Scan während der Navigation hilfreich wäre, so kann er diesen problemlos auch während der Navigation verwenden, da es nicht erforderlich ist, dass Marker in einem solchen Scan abgebildet sind.

Ferner ist insbesondere die Tatsache vorteilhaft, dass ein bereits vorhandenes Kamerasystem durch die erfindungsgemäße Technologie genutzt werden kann, d. h. es werden keine Zusatzgeräte, wie z. B. Laserscanner benötigt. Gegenüber herkömmlichen Verfahren, bei denen das Erfassen bzw. Registrieren von Punkten mit einem Navigationspointer geschieht (d. h. die Knochenoberfläche wird mit einer Pointerspitze abgetastet, die jeweiligen 3D-Koordinaten der Punkte werden gespeichert und diese Punktewolke wird dann zu der aus den CT-Daten gewonnen Oberflächen mathematisch in Deckung gebracht), gestattet das vorliegende erfindungsgemäße Verfahren eine höhere Genauigkeit und schnellere Erfassung.

Die erfindungsgemäße Referenzierung kann für sämtliche Behandlungen verwendet werden, die eine Feststellung der aktuellen Raumlage eines Patientenkörperteils erfordern. Insbesondere eignet sich die Erfindung aber für Verfahren, bei denen die Raumlage der Oberfläche einem zuvor mit einem bildgebenden Verfahren erstellten Bilddatensatz für das Körperteil, insbesondere in einem CT, MRI(Kernspintomographie)-, PET-, SPECT-, Röntgen- oder Ultraschallscan-Datensatz, zugeordnet wird, um die Bilddaten dieses Datensatzes aktuell zu referenzieren.

Grundsätzlich ist es für die Funktion des erfindungsgemäßen Verfahrens vorteilhaft, wenn auf der Oberfläche des Patientenkörperteils ein gut unterscheidbarer Lichtpunkt als Lichtmarkierung erzeugt wird. Der Strahl sollte deshalb möglichst an seinem Auftreffort gut gebündelt sein. Von besonderem Vorteil erweist sich hier die Verwendung eines Lichtstrahles aus unsichtbarem Licht, da sich der damit erzeugte Lichtpunkt sehr gut von den Lichtreflexen unterscheiden lässt, die durch die sonstige Raumbeleuchtung auf dem Patientenkörperteil erzeugt werden. Von Vorteil ist dabei die Verwendung eines Infrarot-Lichtstrahles, wobei die Kameras dann auf die Erfassung von Reflexionen für dieses Licht eingestellt sind. Sehr gut abgegrenzte und gebündelte Lichtmarkierungen lassen sich durch die Verwendung von Laserlicht erhalten.

Wenn, wie oben vorgeschlagen, unsichtbares Licht verwendet wird, um die Lichtmarkierungen zu erzeugen, ist es grundsätzlich schwierig festzustellen, wo der Lichtstrahl gerade aufgestrahlt wird. Um dieses Problem zu lösen wird das erfindungsgemäße Verfahren vorteilhafterweise so durchgeführt, dass durch einen zweiten Lichtstrahl aus sichtbarem Licht, der im Wesentlichen auf denselben Zielbereich gerichtet ist, wie der unsichtbare Referenzierungslichtstrahl, eine sichtbare Lichtreflexion auf der Oberfläche erzeugt wird. Mit Hilfe dieses zweiten Lichtstrahles lässt sich nun sehr gut erkennen, wo der unsichtbare Referenzierungslichtstrahl gerade auftrifft. Auch dieser zweite Lichtstrahl kann ein Laserlichtstrahl sein. Einen weiteren Vorteil bietet die gerade genannte Ausführungsform auch hinsichtlich der Vermeidung von Gefahren. Wie schon oben erwähnt, ist es wegen der Möglichkeit, scharf umrissene Lichtmarkierungen zu erzeugen, von Vorteil, wenn ein unsichtbarer Laserlichtstrahl als Referenzierungslichtstrahl verwendet wird. Da das menschliche Auge diesen Lichtstrahl nicht sehen kann und versehentliche Einstrahlung in das geöffnete Auge keinen Lidschlussreflex erzeugt, können hier Verletzungen hervorgerufen werden, wie z. B. Netzhautverbrennungen. Verwendet man nun, wie gemäß der bevorzugten Ausführungsform vorgeschlagen, einen zweiten Lichtstrahl mit sichtbarem Licht, kann dieser einerseits als Zielhilfe dienen, wobei empfindliche Bereiche (z. B. die Augen) von der Bestrahlung ausgenommen werden. Andererseits bewirkt dieses sichtbare Licht beim Eindringen in das Auge den Lidschlussreflex, so dass Hornhautverbrennungen vermieden werden.

Es gibt grundsätzlich mehrere Möglichkeiten, die beiden Lichtstrahlen zu erzeugen. So können hierzu beispielsweise zwei nebeneinanderliegende Lichtquellen oder zwei ineinanderliegende Lichtquellen verwendet werden. Natürlich besteht auch die Möglichkeit, eine einzige Lichtquelle einzusetzen, die sowohl sichtbares als auch unsichtbares Licht strahlt.

Bei einer bevorzugten Ausführungsform des Verfahrens werden mit dem Referenzierungslichtstrahl auf der Oberfläche nacheinander mehrere Lichtmarkierungen (Punkte) erzeugt, während laufend die Position der erzeugten Lichtmarkierungen erfasst wird, und zwar insbesondere so lange bis eine ausreichende Anzahl von Positionsdaten zur Bestimmung der Raumlage aufgenommen wurde. Es besteht hierbei noch die Möglichkeit, den unterstützenden Computer während der Referenzierung mittels eines Matching-Verfahrens laufend prüfen zu lassen, ob er schon genügend Lichtmarkierungen für die Zuordnung der referenzierten Punktmenge zur Oberfläche aus dem Bilddatensatz (z. B. Tomographie) zur Verfügung hat. Falls dann zu einem bestimmten Zeitpunkt genügend Daten zur Verfügung stehen, kann ein optisches oder akustisches Signal ausgegeben werden, welches anzeigt, dass die Referenzierung erfolgreich war.

Bei einer weiteren vorteilhaften Ausführungsform des erfindungsgemäßen Verfahrens wird das zu referenzierende Körperteil während der Referenzierung so bewegt, dass Kameraschatten eliminiert werden, wobei die Bewegung des Körperteils mittels einer positionsfest gegenüber dem Körperteil angeordneten Markierungsanordnung im Navigationssystem verfolgt wird. Da die Kameras meist fest installiert sind, würden sich insbesondere bei der Referenzierung im Gesicht in bestimmten Lagen Punkte ergeben, die im Kameraschatten liegen, also beispielsweise hinter einem Nasenflügel. Wenn man auch diese Punkte mit Lichtmarkierungen bestrahlen und diese Markierungen erfassen möchte, ist es deshalb vorteilhaft, den Patienten zu bewegen. Damit diese Bewegung die Erfassung nicht verfälscht, muss sie im Navigationssystem verfolgt werden, und dies geschieht mit der genannten Markierungsanordnung, also beispielsweise über einen dreiarmigen Mayfield-Referenzadapter mit einer bekannten Markeranordnung.

Erfindungsgemäß wird ferner eine Vorrichtung zur Referenzierung bzw. Registrierung eines Patienten bzw. eines Körperteils eines Patienten bereitgestellt, mit einem durch mindestens zwei Kameras unterstützten medizinischen Navigationssystem, durch welches computergestützt die dreidimensionalen Raumpositionen von Lichtmarkierungen in einem Erfassungsbereich erfassbar sind, und mit einem Mittel zur Erzeugung von Lichtmarkierungen auf der Oberfläche des zu referenzierenden Körperteils, deren dreidimensionale Raumposition von dem kameragestützten Navigationssystem bestimmbar ist, wobei das Mittel zur Erzeugung der Lichtmarkierungen ein Handlichtstrahler ist, der als Lichtmarkierungen Lichtreflexionen auf der Oberfläche erzeugt. Der Handlichtstrahler kann ein Strahler für unsichtbares Licht, insbesondere Infrarot-Licht sein, wobei die Kameras auf die Erfassung von Reflexionen dieses Lichtes eingestellt sind. Ferner kann der Handlichtstrahler ein Laserlichtstrahler sein.

Bevorzugt strahlt der Handlichtstrahler einen zweiten Lichtstrahl aus sichtbarem Licht ab, der im Wesentlichen auf denselben Zielbereich gerichtet ist, wie der unsichtbare Referenzierungslichtstrahl, wobei zusätzlich eine sichtbare Lichtreflexion auf der Oberfläche erzeugt wird. Auch dieser zweite Lichtstrahler kann sichtbares Laserlicht abstrahlen. Dabei können die Lichtquellen fiir die Lichtstrahlen in einer einzigen Lichtquelle vereint sein, zwei nebeneinanderliegende oder zwei ineinanderliegende Lichtquellen sein.

Vorteilhafterweise umfasst die Vorrichtung noch eine positionsfest gegenüber dem Körperteil angeordnete Markierungsanordnung, mittels der das zu referenzierende Körperteil während einer Bewegung bei der Referenzierung zur Eliminierung von Kameraschatten verfolgt wird.

Umgekehrt kann natürlich auch eine Kamerabewegung verfolgt werden. In den meisten Fällen ist es vorteilhaft, wenn das zu behandelnde Patientenkörperteil während der Behandlung und auch schon während der Referenzierung bzw. Registrierung ruhig fixiert bleibt. In diesem Fall können Kameraschatten vermieden werden, indem die Kameras selbst in ihrer Position verändert werden. Da die Markierungsanordnung aus verschiedenen Kamerawinkeln andere Bilder liefert, kann auch in diesem Fall die Relativbewegung zwischen Kameras und dem Körperteil verfolgt und bei der Referenzierung berücksichtigt werden.

Die mit der erfindungsgemäßen Vorrichtung zu erzielenden Vorteile sind diejenigen, wie sie oben hinsichtlich des Verfahrens gemäß der Erfindung erörtert wurden.

Die Erfindung wird im Weiteren anhand einer Ausführungsform näher erläutert. Hierzu zeigt die einzige beiliegende Figur schematisch die Verwendung einer erfindungsgemäßen Referenzierungsvorrichtung, mittels welcher die Lage eines Patienten bzw. eines Patientenkörperteils referenziert bzw. registriert wird.

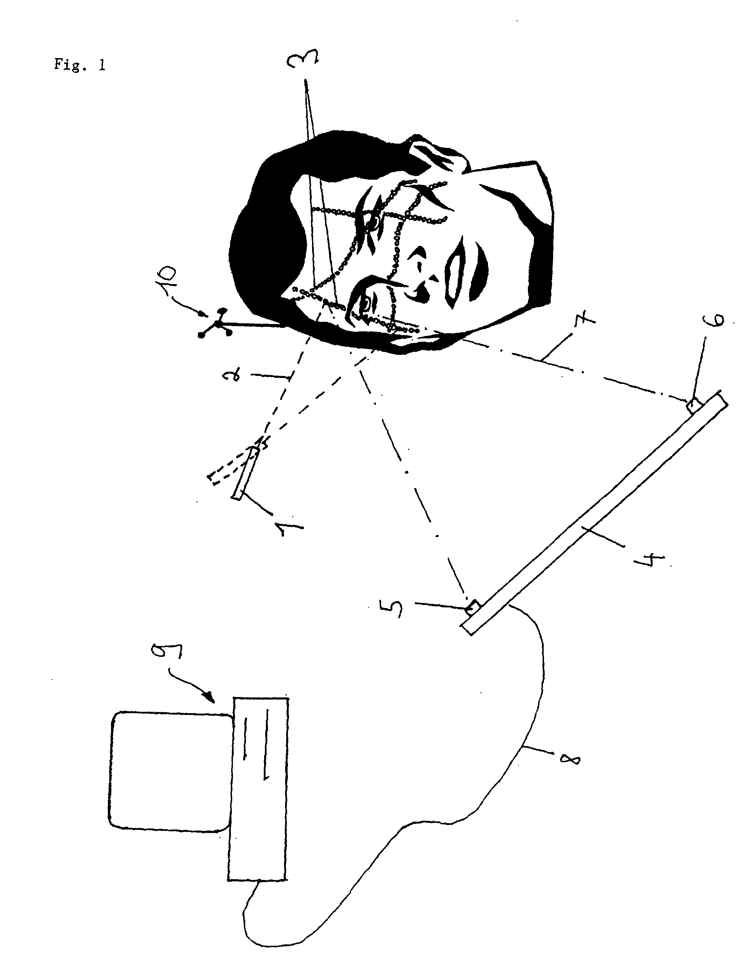

In der Figur ist von dem kameraunterstützten Navigationssystem schematisch der Computer mit Bildschirmausgabe dargestellt und insgesamt mit 9 bezeichnet. Dieser Computer ist über die Kabelverbindung 8 mit dem Kameraträger 4 verbunden, an dem im Abstand zwei Infrarot-Kameras 5 und 6 angebracht sind, welche das Behandlungsgebiet überwachen.

Referenziert bzw. registriert werden soll die Lage des dargestellten menschlichen Kopfes. Hierzu dient der Handlichtstrahler 1, der einen Infrarot-Laserlichtstrahl 2 abstrahlt, und zwar auf die Gesichtsoberfläche des Patienten. Der Handlichtstrahler 1 ist nochmals gestrichelt in einer zweiten Position dargestellt, um darzustellen, dass er während der Referenzierung laufend verschwenkt wird.

Mit dem Referenzierungslichtstrahl 2 wird nunmehr die Gesichtsoberfläche abgefahren, wodurch hintereinander angeordnete Lichtreflexionen bzw. -punkte 3 auf der Oberfläche erzeugt werden. Es sind in der Zeichnung exemplarisch nur einige solcher Lichtmarkierungen dargestellt, und zwar durch in einer Linie aneinandergereihte Lichtpunkte. Die Punkte bzw. Reflexionen können aber generell auch einzeln an geeigneten Orten durch Aufstrahlung erzeugt werden.

Vor der tatsächlichen Behandlung nimmt der Behandelnde ganz einfach den Handlichtstrahler 1 in die Hand und fährt mit dem Lichtstrahl 2 eine zeitlang über die Gesichtsoberfläche. Für das Kamerasystem entstehen dabei wegen der schnell aufeinanderfolgenden Aufnahme von Einzelbildern jeweils hintereinander angeordneten Lichtreflexionen 3, deren Lichtweg für einen Punkt mit der Strichpunktlinie 7 in der Zeichnung dargestellt ist. Die beiden Kameras können die Lage des Lichtreflexes im Raum dreidimensional erfassen und das Computersystem 9 kann aus den Daten der erfassten Lichtmarkierungen dann die Lage von der Oberfläche des Gesichtes zugeordneten Punkten ermitteln.

Im Computer sind die Daten einer Scan-Erfassung für den Patientenkopf gespeichert, und damit auch die Daten für die Gesichtsoberfläche. Der Computer ermittelt nun mit Hilfe eines Matching-Verfahrens laufend, ob ihm die Anzahl der Bildpunkte aus der Referenzierung mittels des Lichtstrahles schon genügt, um die erfassten Oberflächenpunkte der Oberfläche zuzuordnen bzw. damit in Deckung zu bringen, wie sie ihm aus dem Scan-Datensatz bekannt ist. Wenn hierbei eine ausreichende Übereinstimmung vorhanden ist, wird ein akustisches und/oder optisches Signal ausgegeben, das dem Behandelnden anzeigt, dass die Referenzierung erfolgreich abgeschlossen ist.

Die erzeugten Bildpunkte 3 ersetzen also aufgeklebte oder anders angebrachte Markierungen, wie sie bisher separat verwendet wurden. Durch die Vielzahl der erhaltenen Lichtbildpunkte 3 ist es möglich, eine sehr genaue Referenzierung vorzunehmen.

Weiterhin ist in der Figur schematisch dargestellt, dass sich an dem Patientenkopf in fester Positionierung noch ein Referenzadapter 10 befindet. Dieser Adapter weist drei Reflektoren auf, deren Position ebenfalls von den Kameras 5, 6 verfolgt werden kann. Wenn es nunmehr nötig ist, den Kopf des Patienten während der Referenzierung zu drehen oder die Kameras 5,6 zu bewegen, um beispielsweise Kameraschatten hinter einem Nasenflügel zu eliminieren, wird die Relativbewegung mit Hilfe des Adapters 10 verfolgt und bei der Referenzierung mit eingerechnet, so dass es nicht zu Erfassungsfehlem kommt.

Wie schon vorher erwähnt, kann der Handlichtstrahler 1 zusätzlich zu dem unsichtbaren Lichtstrahl 2 noch einen sichtbaren Lichtstrahl in derselben Richtung und mit demselben Focus abstrahlen, um es dem Behandelnden zu gestatten, die erzeugten Lichtpunkte optisch zu verfolgen und Einstrahlungen in die Augen zu vermeiden.

Das erfindungsgemäße Referenzierungssystem ist bei allen Behandlungsmethoden einsetzbar, die eine bildunterstützte Operationstätigkeit mit einbeziehen. Dies gilt sowohl für chirurgische Eingriffe als auch für Bestrahlungsbehandlungen. Die Referenzierung kann für Trackingsysteme mit passiven Markeranordnungen ebenso eingesetzt werden wie für solche mit aktiv abstrahlenden Markern, wie sie zum Beispiel zur Verfolgung von medizinischen Instrumenten verwendet werden. Obwohl bisher meist davon gesprochen wurde, die Lichtmarkierungen mittels des Lichtstrahles auf der Hautoberfläche des Patienten zu erzeugen, ist es im Rahmen der Erfindung durchaus denkbar, auf diese Weise beispielsweise schon zur Behandlung freigelegte Knochenstrukturen zu referenzieren, etwa freigelegte Schädelknochenabschnitte bzw. Wirbelsäulenteile.

## Patentansprüche

1. Verfahren zur Referenzierung bzw. Registrierung eines Patienten bzw. eines Körperteils eines Patienten in einem kameragestützten, medizinischen Navigationssystem mit den folgenden Schritten:
- das zu referenzierende Körperteil des Patienten wird in den Erfassungsbereich eines durch mindestens zwei Kameras (5, 6) unterstützten Navigationssystems gebracht, welches computergestützt die dreidimensionalen Raumpositionen von Lichtmarkierungen (3) erfasst,
- mittels eines Lichtstrahles (2) aus einem Handlichtstrahler (1), der nicht mittels des Navigationssystems positionell verfolgt wird, werden auf der Oberfläche des zu referenzierenden Körperteils Lichtmarkierungen (3) erzeugt, deren dreidimensionale Position von allein den vorhandenen Kameras des kameragestützten Navigationssystem bestimmt wird,
- mittels der Positionsdaten für die Lichtmarkierungen (3) wird die Raumlage der Oberfläche des zu referenzierenden Körperteils bestimmt.

2. Verfahren nach Anspruch 1, bei dem die Raumlage der Oberfläche einem zuvor mit einem bildgebenden Verfahren erstellten Bilddatensatz für das Körperteil, insbesondere einem CT-, MRI(Kernspintomographie)-, PET-, SPECT-, Röntgen- oder Ultraschallscan-Datensatz zugeordnet wird, um die Bilddaten dieses Datensatzes aktuell zu referenzieren.

3. Verfahren nach einem der Ansprüche 1 oder 2, bei dem der Lichtstrahl ein Strahl unsichtbaren Lichtes, insbesondere Infrarotlichtes ist, wobei die Kameras (5, 6) auf die Erfassung von Reflexionen für dieses Licht eingestellt sind.

4. Verfahren nach Anspruch 3, bei dem der Lichtstrahl ein Laserlichtstrahl ist.

5. Verfahren nach Anspruch 3 oder 4, bei dem durch einen zweiten Lichtstrahl aus sichtbarem Licht, der im Wesentlichen auf denselben Zielbereich gerichtet ist, wie der unsichtbare Referenzierungslichtstrahl (2) eine sichtbare Lichtreflexion auf der Oberfläche erzeugt wird.

6. Verfahren nach Anspruch 5, bei dem der zweite Lichtstrahl ein sichtbarer Laserstrahl ist.

7. Verfahren nach Anspruch 6 oder 7, bei dem die beiden Lichtstrahlen mit einer einzigen, zwei nebeneinanderliegenden oder zwei ineinanderliegenden Lichtquellen erzeugt werden.

8. Verfahren nach einem der Ansprüche 1 bis 7, bei dem mit dem Referenzierungslichtstrahl (2) auf der Oberfläche nacheinander mehrere Lichtmarkierungen erzeugt werden, während laufend die Position der erzeugten Lichtmarkierungen (3) erfasst wird, und zwar insbesondere so lange bis eine ausreichende Anzahl von Positionsdaten zur Bestimmung der Raumlage aufgenommen wurde.

9. Verfahren nach einem der Ansprüche 1 bis 8, bei dem entweder die Kameraanordnung oder das zu referenzierende Körperteil während der Referenzierung so bewegt wird, dass Kameraschatten eliminiert werden, wobei eine Relativbewegung von Körperteil und Kameraanordnung mittels einer positionsfest gegenüber dem Körperteil angeordneten Markierungsanordnung (10) im Navigationssystem verfolgt wird.

10. Vorrichtung zur Referenzierung bzw. Registrierung eines Patienten bzw. eines Körperteils eines Patienten mit einem durch mindestens zwei Kameras (5, 6) unterstützten medizinischen Navigationssystem, durch welches computergestützt die dreidimensionalen Raumpositionen von Lichtmarkierungen (3) in einem Erfassungsbereich erfassbar sind, und einem Mittel zur Erzeugung von Lichtmarkierungen (3) auf der Oberfläche des zu referenzierenden Körperteils, deren dreidimensionale Raumposition von dem kameragestützten Navigationssystem bestimmbar ist, wobei das Mittel zur Erzeugung der Lichtmarkierungen (3) ein Handlichtstrahler (1) ist, der als Lichtmarkierungen Lichtreflexionen auf der Oberfläche erzeugt,
**dadurch gekennzeichnet, dass**
die dreidimensionmale Raumposition der Lichtmarkierungen (3) von den vorhandenen Kameras des Navigationssystems bestimmt werden kann, ohne dass der Handlichtstrahler mittels des Navigationssystems positionell verfolgt werden muß.

11. Vorrichtung nach Anspruch 10, **dadurch gekennzeichnet, dass** der Handlichtstrahler (1) ein Strahler für unsichtbares Licht, insbesondere Infrarotlicht ist, wobei die Kameras (5, 6) auf die Erfassung von Reflexionen für dieses Licht eingestellt sind.

12. Vorrichtung nach Anspruch 11, **dadurch gekennzeichnet, dass** der Handlichtstrahler (1) ein Laserlichtstrahler ist.

13. Vorrichtung nach Anspruch 11 oder 12, **dadurch gekennzeichnet, dass** der Handlichtstrahler (1) einen zweiten Lichtstrahl aus sichtbarem Licht abstrahlt, der im Wesentlichen auf denselben Zielbereich gerichtet ist, wie der unsichtbare Referenzierungslichtstrahl, wobei zusätzlich eine sichtbare Lichtreflektion auf der Oberfläche erzeugt wird.

14. Vorrichtung nach Anspruch 13, **dadurch gekennzeichnet, dass** der zweite Lichtstrahler einer für sichtbares Laserlicht ist.

15. Vorrichtung nach Anspruch 13 oder 14, **dadurch gekennzeichnet, dass** die Lichtquellen für die beiden Lichtstrahlen in einer einzigen Lichtquelle vereint sind, zwei nebeneinanderliegende oder zwei ineinanderliegende Lichtquellen sind.

16. Vorrichtung nach einem der Ansprüche 10 bis 15, **dadurch gekennzeichnet, dass** sie ferner eine positionsfest gegenüber dem Körperteil angeordnete Markierungsanordnung (10) umfasst, mittels der eine Relativbewegung zwischen dem zu referenzierenden Körperteil und der Kameraanordnung zur Eliminierung von Kameraschatten bei der Referenzierung verfolgt wird.

## Claims

1. A method for referencing or registering a patient or a patient body part in a camera-assisted, medical navigation system comprising the following steps:
- the patient body part to be referenced is brought into the detecting range of a navigation system assisted by at least two cameras (5, 6), this navigation system detecting with computer support the three-dimensional, spatial positions of light marks (3),
- light marks (3) are generated on the surface of the part of the body to be referenced by means of a light beam (2) from a manual light beamer (1) which is not positionally tracked by means of the navigation system, the three-dimensional position of the light marks (3) being determined solely by the available cameras of the camera-assisted navigation system,
- the three-dimensional position of the surface of the part of the body to be referenced is determined by means of the positional data for the light marks (3).

2. The method as set forth in claim 1, wherein the spatial position of said surface is assigned to a set of image data, previously produced by an imaging technique, for said part of the body concerned, especially a CT, MRI (magnetic nuclear resonance tomograph), PET, SPECT, x-ray or ultrasound scan data set to update-reference said image data of this data set.

3. The method as set forth in claim 1 or 2, wherein said light beam is a beam of invisible light, in particular infrared light, said cameras (5, 6) being set to detect the reflections for this light.

4. The method as set forth in claim 3, wherein said light beam is a laser light beam.

5. The method as set forth in claim 3 or 4, wherein by means of a second beam of visible light, aimed substantially at the same target area as that of said invisible referencing light beam (2), a visible light reflection is created on said surface.

6. The method as set forth in claim 5, wherein said second light beam is a visible laser beam.

7. The method as set forth in claim 6 or 7, wherein said two light beams are generated by two light sources located juxtaposed or nested.

8. The method as set forth in any of the claims 1 to 7, wherein several light marks are generated in sequence on said surface by said referencing light beam (2), while the position of said generated light marks (3) is detected all the time, i.e. in particular until a sufficient number of positional data for determining said spatial position has been acquired.

9. The method as set forth in any of the claims 1 to 8, wherein either the camera arrangement or said body part to be referenced is moved during referencing so that camera shades are eliminated, a relative movement of said body part being tracked in said navigation system by means of a marker array (10) fixedly positioned relative to said body part.

10. An apparatus for referencing or registering a patient or patient body part, including a medical navigation system assisted by at least two cameras (5, 6), by which the three-dimensional, spatial positions of light marks (3) may be detected in a detection area with computer support, and means for generating said light marks (3) on the surface of said body part to be referenced, the three-dimensional, spatial position of said light marks (3) being determinable by said camera-assisted navigation system, whereby said means for generating said light marks (3) is a manual light beamer (1) producing light reflections on said surface as light marks,
**characterised in that**
the three-dimensional, spatial position of said light marks (3) may be determined by the available cameras of said camera-assisted navigation system, without said manual light beamer (1) having to be positionally tracked by means of said navigation system.

11. The apparatus as set forth in claim 10, **characterised in that** said manual light beamer (1) is a beamer for invisible light, in particular infrared light, said cameras (5, 6) being set to capture the reflections of said light.

12. The apparatus as set forth in claim 11, **characterised in that** said manual light beamer (1) is a laser manual light beamer.

13. The apparatus as set forth in claim 11 or 12, **characterised in that** said manual light beamer (1) beams a second beam of visible light, aimed substantially at said same target area as that of said invisible referencing light beam, a visible light reflection being generated in addition on said surface.

14. The apparatus as set forth in claim 13, **characterised in that** said second manual light beamer is a beamer for visible laser light.

15. The apparatus as set forth in claim 13 or 14, **characterised in that** the light sources for said beams are unified into a single light source or are two juxtaposed or two nested light sources.

16. The apparatus as set forth in any of claims 10 to 15, **characterised in that** it comprises a marker array (10), fixedly positioned relative to said body part, by means of which a relative movement between said body part to be referenced and said camera arrangement is tracked to eliminate camera shades during referencing.

## Revendications

1. Procédé pour le référencement ou l'enregistrement d'un patient ou d'une partie du corps d'un patient dans un système de navigation médicale assistée par caméra, comprenant les étapes suivantes:
- la partie du corps du patient à référencer est placée dans la zone de saisie d'un système de navigation assisté par au moins deux caméras (5, 6), qui saisit avec l'aide d'un ordinateur les positions spatiales tridimensionnelles de repères lumineux (3),
- au moyen d'un rayon lumineux émis par un émetteur de lumière (1) tenu à la main, dont la position n'est pas suivie par le système de navigation, des repères lumineux (3) dont la position tridimensionnelle est déterminée uniquement par les caméras prévues dans le système de navigation assistée par caméras sont créés à la surface de la partie du corps à référencer,
- la position spatiale de la surface de la partie du corps à référencer est déterminée au moyen des données de position des repères lumineux (3).

2. Procédé selon la revendication 1, dans lequel la position spatiale de la surface est associée à un ensemble de données d'image déterminées préalablement par un procédé de création d'image, en particulier à un ensemble de données de numérisation par CT, MRI (tomographie par spin de noyaux), PET, SPECT, rayons X ou ultrasons, pour se référer effectivement aux données d'image de cet ensemble de données.

3. Procédé selon l'une des revendications 1 ou 2, dans lequel le rayon lumineux est un rayon de lumière invisible, en particulier de lumière infrarouge, les caméras (5, 6) étant utilisées pour saisir les réflexions de cette lumière.

4. Procédé selon la revendication 3, dans lequel le rayon lumineux est un rayon de lumière laser.

5. Procédé selon la revendication 3 ou 4, dans lequel une réflexion de lumière visible est créée à la surface par un deuxième rayon lumineux de lumière visible qui est dirigé essentiellement sur la même zone cible que le rayon lumineux invisible de référencement (2).

6. Procédé selon la revendication 5, dans lequel le deuxième rayon lumineux est un rayon de lumière laser visible.

7. Procédé selon la revendication 6 ou 7, dans lequel les deux rayons lumineux sont créés par une seule source de lumière, deux sources de lumière situées l'une à côté de l'autre ou deux sources de lumière situées l'une dans l'autre.

8. Procédé selon l'une des revendications 1 à 7, dans lequel plusieurs repères lumineux sont créés successivement à la surface par le rayon lumineux de référencement (2), la position des repères lumineux (3) étant saisie en permanence jusqu'à ce qu'un nombre suffisant de données de position ait été enregistré pour la détermination de la position.

9. Procédé selon l'une des revendications 1 à 8, dans lequel l'agencement de caméras ou la partie du corps à référencer sont déplacés de manière à éliminer l'ombre des caméras, un déplacement relatif de la partie du corps et de l'agencement de caméras étant suivi au moyen d'un agencement de repérage (10), disposé en position fixe par rapport à la partie du corps, du système de navigation.

10. Dispositif pour le référencement ou l'enregistrement d'un patient ou d'une partie du corps d'un patient, comportant un système de navigation médicale assisté par au moins deux caméras (5, 6) par lequel les positions spatiales tridimensionnelles de repères lumineux (3) peuvent être saisies dans une zone de saisie, et un moyen pour la création à la surface de la partie du corps à référencer de repères lumineux (3) dont la position spatiale tridimensionnelle peut être déterminée par le système de navigation assisté par caméras, dans lequel :
- le moyen pour créer les repères lumineux (3) est un émetteur de lumière (1) tenu à la main qui crée à la surface des réflexions de lumière qui servent de repères lumineux,
**caractérisé en ce que** la position spatiale tridimensionnelle des repères lumineux (3) peut être déterminée par les caméras prévues dans le système de navigation sans que la position de l'émetteur de lumière tenu à la main doive être suivie par le système de navigation.

11. Dispositif selon la revendication 10, **caractérisé en ce que** l'émetteur de lumière (1) tenu à la main est un émetteur de lumière invisible, en particulier de lumière infrarouge, les caméras (5, 6) étant utilisées pour saisir les réflexions de cette lumière.

12. Dispositif selon la revendication 11, **caractérisé en ce que** l'émetteur de lumière (1) tenu à la main est un émetteur de lumière laser.

13. Dispositif selon la revendication 11 ou 12, **caractérisé en ce que** l'émetteur de lumière (1) tenu à la main émet un deuxième rayon lumineux de lumière visible qui est dirigé essentiellement sur la même zone cible que le rayon lumineux invisible de référence, une réflexion de lumière étant en même temps créée à la surface.

14. Dispositif selon la revendication 13, **caractérisé en ce que** le deuxième émetteur de lumière est un émetteur de lumière visible.

15. Dispositif selon la revendication 13 ou 14, **caractérisé en ce que** les sources de lumière prévues pour les deux rayons lumineux sont réunies en une seule source de lumière, en deux sources de lumières situées l'une à côté de l'autre ou en deux sources de lumières situées l'une dans l'autre.

16. Dispositif selon l'une des revendications 10 à 15, **caractérisé en ce qu'**il comporte en outre un agencement de repérage (10) qui est disposé en position fixe par rapport à la partie du corps et qui permet de suivre un déplacement relatif entre la partie du corps à référencer et l'agencement de caméras pour éliminer l'ombre des caméras lors du référencement.
